# EUROPEAN PATENT APPLICATION

(11) **EP 4 810 063 A1**
(43) Date of publication of application: **23.09.2026**
(21) Application number: 24890732.1
(22) Date of filing: 13.11.2024
(51) Int. Cl.: A61K 47/68, A61K 31/4745, A61P 35/00

(54) **METHOD FOR TREATING CANCER VIA ANTI-B7H3 ANTIBODY-DRUG CONJUGATE**

(30) Priority: 13.11.2023 CN 202311501263; 07.08.2024 CN 202411076978
(71) Applicant: Medilink Therapeutics (Suzhou) Co., Ltd., Suzhou, Jiangsu 215000 (CN)
(72) Inventor: XUE, Tongtong, Suzhou, Jiangsu 215000 (CN); LIAN, Wei, Suzhou, Jiangsu 215000 (CN); CAI, Jiaqiang, Suzhou, Jiangsu 215000 (CN); CHIN, Steve, Suzhou, Jiangsu 215000 (CN); WANG, Ruihua, Suzhou, Jiangsu 215000 (CN); ZHANG, Xian, Suzhou, Jiangsu 215000 (CN); XIAO, Liang, Suzhou, Jiangsu 215000 (CN)
(74) Representative: Mewburn Ellis LLP
(86) International application number: PCT/CN2024/131839
(87) International publication number: WO 2025/103377

(57) **Abstract**

A method or use related to administering an anti-B7H3 antibody-drug conjugate in combination with another therapeutic agent to treat cancer; said other therapeutic agent is selected from anti-PD-L1 antibody or antigen-binding fragment thereof or/and a chemotherapy drug. The present invention further relates to the use of a drug composition and a drug kit in the treatment of cancer.

## Description

The present application claims priority to the Chinese patent application 2023115012636 filed on November 13, 2023 and the Chinese patent application 2024110769786 filed on August 7, 2024. The above-mentioned Chinese patent applications are incorporated herein by reference in their entirety.

### TECHNICAL FIELD

The present invention pertains to the technical field of molecular immunology and tumour treatment. The present invention relates to a method or use related to administering an anti-B7H3 antibody-drug conjugate in combination with an additional therapeutic agent to treat cancer; the additional therapeutic agent is selected from an anti-PD-L1 antibody or antigen-binding fragment thereof or/and a chemotherapy drug. The present invention further relates to the use of the combination in a pharmaceutical composition and a drug kit used in the treatment of cancer.

### BACKGROUND ART

Malignant tumours have become a major global public health problem, resulting in nearly 10 million deaths in 2020. The number of cancer patients and the number of deaths due to cancer have continued to rise globally, resulting in the continuous expansion of the overall cancer treatment market and an increasing demand for new therapeutic drugs and treatment modalities.

Monoclonal antibody drugs have the advantages of strong targeting, high specificity and a low incidence of adverse reactions, and antibody-drug conjugates (ADC) are representative products among those drugs. Since ADC possesses both the efficacy of small molecule drugs and the targeting properties of antibody drugs, it can reduce the toxicity of cytotoxic drugs while enhancing therapeutic effects. At present, 15 ADC drugs have been approved for marketing worldwide for indications including breast cancer, lung cancer and gastrointestinal cancers. From the perspective of clinical effects, when ADC drugs are used to treat solid tumours, the response rate for solid tumour types is relatively low. For example, Trodelvy^{®} is an ADC drug targeting Trop-2, and in the treatment of triple-negative breast cancer (TNBC), the objective response rate (ORR) is 27% to 35%; Tivdak^{®} is an ADC drug targeting TF, and in the treatment of recurrent or malignant cervical cancer, the ORR is 24%. At the same time, since ADC is conjugated with cytotoxic compound payloads, toxicities are observed in clinical use, such as haematological adverse reactions, including pancytopenia, neutropenia, thrombocytopenia, etc., as well as peripheral neuropathy, hepatotoxicity, pulmonary toxicity, cardiotoxicity, etc., thereby limiting clinical dosing and resulting in suboptimal therapeutic effects in certain indications. Accordingly, additional treatment modalities for ADC drugs, for example use in combination with drugs having different mechanisms of action, remain to be explored in order to further improve therapeutic effects.

Immune checkpoints, as immunosuppressive pathways, are crucial for maintaining self-tolerance and regulating the duration and extent of immune responses in peripheral tissues. However, these pathways can be "hijacked" by tumours and continuously activated, suppressing anti-tumour immunity and promoting tumour development (Pardoll DM. The blockade of immune checkpoints in cancer immunotherapy. Nat Rev Cancer. 2012;12(4):252-264); Haanen JB, Robert C. Immune Checkpoint Inhibitors. Prog Tumor Res. 2015;42:55-66). Programmed death-1 (PD-1) and its ligand (PD-L1) are the most widely applied immune checkpoint inhibitor (ICI) targets at present and are approved for the clinical treatment of multiple tumours, but due to the heterogeneity of tumours and the complexity of the tumour microenvironment, the overall efficacy of immune checkpoint inhibitor treatment is relatively low (Taube JM, Klein A, Brahmer JR, et al. Association of PD-1, PD-1 ligands, and other features of the tumour immune microenvironment with response to anti-PD-1 therapy. Clin Cancer Res. 2014;20(19):5064-5074; Restifo NP, Smyth MJ, Snyder A. Acquired resistance to immunotherapy and future challenges. Nat Rev Cancer. 2016;16(2):121-126), and for most cancer types, only 20% to 30% of patients exhibit an immune response.

Small-molecule cytotoxic drugs have been used clinically for many years and are first-line treatments for a variety of diseases. Although such drugs may show significant efficacy at an early stage of treatment, they are highly toxic, poorly tolerated during long-term administration, and prone to inducing drug resistance.

### SUMMARY OF THE INVENTION

In view of the shortcomings of immune checkpoint inhibitors and small-molecule cytotoxic agents in the treatment of cancer, the present disclosure provides a combination of an antibody-drug conjugate and additional therapeutic agent(s), with a view to achieving excellent anti-tumour effects in the treatment of cancer in an individual, such as a human or animal, for example enhanced efficacy, increased durability of therapeutic response and/or reduced dose-dependent toxicity.

In a first aspect, the present disclosure provides a method of treating cancer, the method comprising administering to a subject, for example to a subject in need thereof, an effective amount of:
(a) an anti-B7H3 antibody-drug conjugate, which comprises an anti-B7H3 antibody or antigen-binding fragment thereof and a camptothecin drug, wherein the anti-B7H3 antibody or antigen-binding fragment thereof comprises a heavy chain variable region and a light chain variable region, wherein:
   the heavy chain variable region comprises:
      (i) CDR-H1 comprising the amino acid sequence set forth in SEQ ID NO: 1;
      (ii) CDR-H2 comprising the amino acid sequence set forth in SEQ ID NO: 2; and
      (iii) CDR-H3 comprising the amino acid sequence set forth in SEQ ID NO: 3; and
   the light chain variable region comprises:
      (i) CDR-L1 comprising the amino acid sequence set forth in SEQ ID NO: 4;
      (ii) CDR-L2 comprising the amino acid sequence set forth in SEQ ID NO: 5; and
      (iii) CDR-L3 comprising the amino acid sequence set forth in SEQ ID NO: 6;
   wherein the CDRs are determined in accordance with the Kabat definition scheme;
and (b) an additional therapeutic agent, wherein the additional therapeutic agent is selected from an anti-PD-L1 antibody or antigen-binding fragment thereof, a chemotherapy drug, or one, two or more of any of the foregoing.

In some embodiments, the anti-B7H3 antibody or antigen-binding fragment thereof of the anti-B7H3 antibody-drug conjugate comprises a heavy chain variable region and a light chain variable region, wherein the heavy chain variable region comprises an amino acid sequence having at least 85% sequence identity to the amino acid sequence set forth in SEQ ID NO: 7, and the light chain variable region comprises an amino acid sequence having at least 85% sequence identity to the amino acid sequence set forth in SEQ ID NO: 8.

In some embodiments, the amino acid sequence of the heavy chain variable region is an amino acid sequence having at least 85% sequence identity to SEQ ID NO: 7, and the amino acid sequence of the light chain variable region is an amino acid sequence having at least 85% sequence identity to SEQ ID NO: 8.

In some embodiments, the heavy chain variable region of the anti-B7H3 antibody or antigen-binding fragment thereof comprises an amino acid sequence set forth in SEQ ID NO: 7, and the light chain variable region comprises an amino acid sequence set forth in SEQ ID NO: 8.

In some embodiments, the amino acid sequence of the heavy chain variable region of the anti-B7H3 antibody or antigen-binding fragment thereof is set forth in SEQ ID NO: 7, and the amino acid sequence of the light chain variable region is set forth in SEQ ID NO: 8.

In some embodiments, the anti-B7H3 antibody comprises a heavy chain set forth in SEQ ID NO: 9, and a light chain set forth in SEQ ID NO: 10.

In some embodiments, the anti-B7H3 antibody comprises a heavy chain having an amino acid sequence having at least 85% sequence identity to SEQ ID NO: 9, and a light chain having an amino acid sequence having at least 85% sequence identity to SEQ ID NO: 10.

In some embodiments, the anti-B7H3 antibody or antigen-binding fragment thereof is the antibody designated 2E3-02 in WO2022170971.

In a second aspect, the present disclosure provides a method of treating cancer, the method comprising administering to a subject, for example to a subject in need thereof, an effective amount of:
a) an anti-B7H3 antibody-drug conjugate, wherein the anti-B7H3 antibody-drug conjugate is an antibody-drug conjugate having the following formula, a pharmaceutically acceptable salt, stereoisomer or metabolite thereof, or a solvate of any of the foregoing:

   Tb-(L-D)q,

   wherein:
   Tb is an anti-B7H3 antibody or antigen-binding fragment thereof; preferably, Tb is the anti-B7H3 antibody or antigen-binding fragment thereof according to the first aspect described above; L is a linker and has the structure as the following formula: 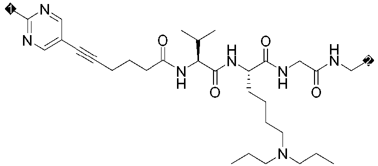
   position 1 is linked to Tb, and position 2 is linked to D;
   D is a bioactive molecular fragment, for example a camptothecin drug;
   q is selected from any numerical value ranging from 0.1 to 16.0; preferably, q is selected from 1, 2, 3, 4, 5, 6, 7, 8, 9 and 10; more preferably, q is 2, 4, 6 or 8;
and (b) an additional therapeutic agent, wherein the additional therapeutic agent is selected from an anti-PD-L1 antibody or antigen-binding fragment thereof, a chemotherapy drug, or one, two or more of any of the foregoing.

In some embodiments, the anti-B7H3 antibody-drug conjugate is an antibody-drug conjugate having the structure shown in the following formula I, a pharmaceutically acceptable salt, stereoisomer or metabolite thereof, or a solvate of any of the foregoing: 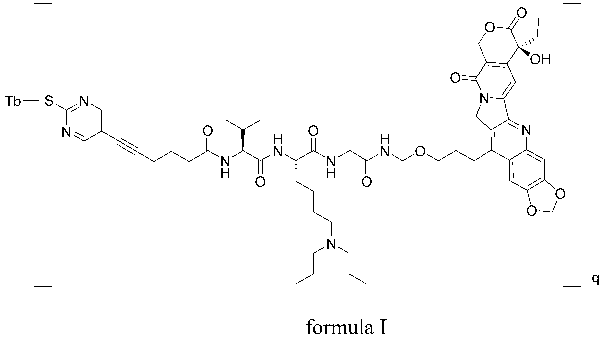 wherein:
S is a sulphur atom on Tb;
Tb is an anti-B7H3 antibody or antigen-binding fragment thereof;
q is selected from any numerical value ranging from 0.1 to 16.0; preferably, q is selected from 1, 2, 3, 4, 5, 6, 7, 8, 9, 10; more preferably, q is 2, 4, 6 or 8.

In some other embodiments, the antibody-drug conjugate may further be selected from: 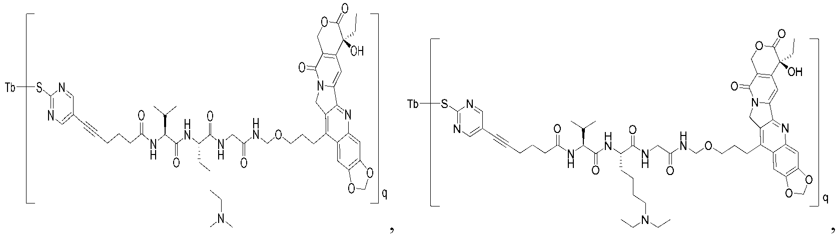 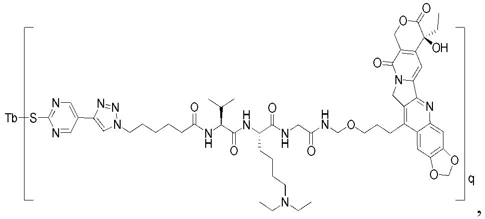 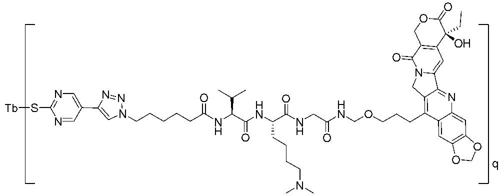 and 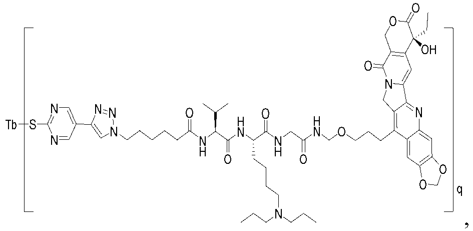
wherein Tb is an anti-B7H3 antibody or antigen-binding fragment thereof;
S is a sulphur atom on Tb;
q is a drug-antibody coupling ratio, and is selected from any numerical value ranging from 0.1 to 16.0, preferably, 1 to 10; preferably, q is 1, 2, 3, 4, 5, 6, 7 or 8; more preferably, q is 2, 4, 6 or 8.

In some embodiments, the anti-B7H3 antibody-drug conjugate is an antibody-drug conjugate having the structure shown in the following formula II, a pharmaceutically acceptable salt, stereoisomer or metabolite thereof, or a solvate of any of the foregoing, 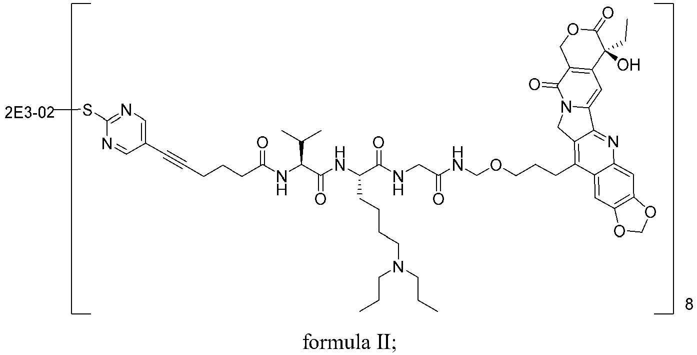 wherein S is a sulphur atom on Tb.

In some embodiments, the anti-B7H3 antibody or antigen-binding fragment thereof as well as the monoclonal antibody or antigen-binding fragment thereof comprise: Fab, Fab', F(ab')₂, Fd, Fv (for example, scFv), dAb, a complementarity-determining region fragment, a non-human antibody, a humanised antibody, a chimeric antibody, a fully human antibody, a probody, a monoclonal antibody, a bispecific antibody or a multispecific antibody.

In some embodiments, Tb is an antibody having B7H3-2Ig and/or B7H3-4Ig or an antigen-binding fragment thereof.

In some embodiments, Tb is an antibody having B7H3-4Ig binding activity higher than B7H3-2Ig binding activity or an antigen-binding fragment thereof.

In some embodiments, Tb is a non-human antibody, a humanised antibody, a chimeric antibody or a fully human antibody.

In some embodiments, Tb is a monoclonal antibody, a bispecific antibody or a multispecific antibody.

In some embodiments, Tb is a monoclonal antibody or antigen-binding fragment thereof.

In some embodiments, the anti-B7H3 antibody or antigen-binding fragment thereof is the antibodies designated 1D1, 1D1-01, 2E3, 2E3-02 in WO2022170971, enoblituzumab, mirzotamab, omburtamab, the antibody designated M30-H1-L4 in CN 103687945B, the antibody designated mAb-C-DUBA in CN 109069633A, and the anti-B7H3 antibody or antigen-binding fragment thereof with reference to CN112521512, WO2021027674, WO2021021543, WO2021006619, CN111662384, CN111454357, WO2020151384, WO2020140094, WO2020103100, WO2020102779, WO2020063673, WO2020047257, WO2020041626, CN110684790, CN110642948, WO2019225787, WO2019226017, US20190338030, CN110305213, WO2018209346, WO2018177393, US9150656, WO2016106004, WO2016044383, WO2016033225, WO2015181267, US20120294796, WO2011109400, CN101104639, WO2004093894, WO2002010187 or WO2001018021.

In some embodiments, the anti-B7H3 antibody or antigen-binding fragment thereof is the antibodies designated 1D1, 1D1-01, 2E3 or 2E3-02 in WO2022170971; more preferably, the anti-B7H3 antibody is the antibody designated 1D1-01 or 2E3-02; further preferably, the B7H3 antibody is the antibody designated 2E3-02.

In some embodiments, the anti-B7H3 antibody is the 2E3-02 antibody, and the 2E3-02 antibody comprises a heavy chain set forth in SEQ ID NO: 9 and a light chain set forth in SEQ ID NO: 10.

In some embodiments, the antibody or antigen-binding fragment further comprises a framework region derived from human or murine immunoglobulin.

In some embodiments, the additional therapeutic agent is an anti-PD-L1 antibody or antigen-binding fragment thereof.

In some embodiments, the additional therapeutic agent is an anti-PD-L1 antibody or antigen-binding fragment thereof and a chemotherapy drug.

In some embodiments, the additional therapeutic agent satisfies the following one or two conditions:
i.the anti-PD-L1 antibody or antigen-binding fragment thereof is selected from: durvalumab, atezolizumab, envafolimab, sugemalimab, adebrelimab, tagitanlimab, socazolimab, benmelstobart, or a combination of any of the foregoing; the anti-PD-L1 antibody may also be the Bio X Cell BO0101 antibody;
ii.the chemotherapy drug is selected from platinum-based drugs; preferably, the platinum-based chemotherapy drugs are selected from cisplatin, carboplatin, sulfatodiaminocyclohexane platin, nedaplatin, oxaliplatin, lobaplatin, satraplatin, miboplatin, enloplatin, iproplatin, dicycloplatin, or a combination of any of the foregoing;
iii.the anti-PD-1 antibody is not durvalumab;
iv.the anti-PD-1 antibody is not atezolizumab.

In some preferred embodiments, the anti-PD-L1 antibody or antigen-binding fragment thereof is selected from: durvalumab, atezolizumab, and the Bio X Cell BO0101 antibody. In some preferred embodiments, the anti-PD-L1 antibody or antigen-binding fragment thereof is selected from: durvalumab and atezolizumab.

In some embodiments, the method comprises administering to the subject an anti-B7H3 antibody-drug conjugate and an anti-PD-L1 antibody or antigen-binding fragment thereof.

In some embodiments, the method comprises administering to the subject an anti-B7H3 antibody-drug conjugate, an anti-PD-L1 antibody or antigen-binding fragment thereof, and a platinum-based drug.

In some embodiments, the platinum-based drug is selected from carboplatin.

In some embodiments, the platinum-based drug is selected from cisplatin.

In some embodiments, the method of treating cancer comprises administering to the subject:
(a) an anti-B7H3 antibody-drug conjugate, wherein the anti-B7H3 antibody-drug conjugate is an antibody-drug conjugate having the structure shown in the following formula I, a pharmaceutically acceptable salt, stereoisomer or metabolite thereof, or a solvate of any of the foregoing: 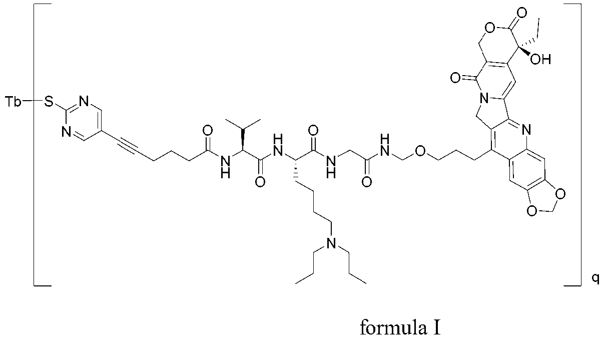 wherein: S is a sulphur atom on Tb; Tb is an anti-B7H3 antibody or antigen-binding fragment thereof; q is selected from any numerical value ranging from 0.1 to 16.0; preferably, q is selected from 1, 2, 3, 4, 5, 6, 7, 8, 9, 10; more preferably, q is 2, 4, 6 or 8; still more preferably, q is 8;
   the anti-B7H3 antibody or antigen-binding fragment thereof comprises a heavy chain variable region and a light chain variable region, wherein:
   the heavy chain variable region (VH) comprises:
      (i) CDR-H1 comprising the amino acid sequence set forth in SEQ ID NO: 1;
      (ii) CDR-H2 comprising the amino acid sequence set forth in SEQ ID NO: 2; and
      (iii) CDR-H3 comprising the amino acid sequence set forth in SEQ ID NO: 3; and
   the light chain variable region (VL) comprises:
      (i) CDR-L1 comprising the amino acid sequence set forth in SEQ ID NO: 4;
      (ii) CDR-L2 comprising the amino acid sequence set forth in SEQ ID NO: 5; and
      (iii) CDR-L3 comprising the amino acid sequence set forth in SEQ ID NO: 6;
   wherein the CDRs are determined in accordance with the Kabat definition scheme;
and (b) an anti-PD-1 antibody or antigen-binding fragment thereof, and the anti-PD-1 antibody is atezolizumab.

In some embodiments, the method of treating cancer further comprises administering to the subject: (c) a platinum-based drug. In some embodiments, the platinum-based drug is selected from carboplatin. In some embodiments, the platinum-based drug is selected from cisplatin.

In some embodiments, the anti-B7H3 antibody-drug conjugate, the pharmaceutically acceptable salt, stereoisomer or metabolite thereof, or the solvate of any of the foregoing is administered at a dose of 0.1-15 mg/kg individual body weight.

In some embodiments, the anti-B7H3 antibody-drug conjugate is administered to the subject at a dose within the following ranges: 0.1 to 10 mg/kg, 0.2 to 8 mg/kg, 0.3 to 6 mg/kg, 0.4 to 4 mg/kg, or 0.5 to 3 mg/kg;
in some embodiments, the anti-B7H3 antibody-drug conjugate is administered to the subject at a dose as follows: 0.4 mg/kg, 0.5 mg/kg, 0.6 mg/kg, 0.8 mg/kg, 1.0 mg/kg, 1.2 mg/kg, 1.4 mg/kg, 1.6 mg/kg, 1.8 mg/kg, 2.0 mg/kg, 2.2 mg/kg, 2.4 mg/kg, 2.6 mg/kg, 2.8 mg/kg, 3.0 mg/kg, 4.0 mg/kg, 5.0 mg/kg.

In some embodiments, the anti-B7H3 antibody-drug conjugate is administered to the subject at a dose of about 1.2 mg/kg.

In some embodiments, the anti-B7H3 antibody-drug conjugate is administered to the subject at a dose of about 1.4 mg/kg.

In some embodiments, the anti-B7H3 antibody-drug conjugate is administered to the subject at a dose of about 1.6 mg/kg.

In some embodiments, the anti-B7H3 antibody-drug conjugate is administered to the subject at a dose of about 1.8 mg/kg.

In some embodiments, the anti-B7H3 antibody-drug conjugate is administered to the subject at a dose of about 2.0 mg/kg.

In some embodiments, the anti-B7H3 antibody-drug conjugate is administered to the subject at a dose of about 2.2 mg/kg.

In some embodiments, the anti-B7H3 antibody-drug conjugate is administered to the subject at a dose of about 2.4 mg/kg.

In some embodiments, the anti-B7H3 antibody-drug conjugate is administered to the subject at a dose of about 2.6 mg/kg.

In some embodiments, the anti-B7H3 antibody-drug conjugate is administered to the subject at a dose of about 2.8 mg/kg.

In some embodiments, the anti-B7H3 antibody-drug conjugate is administered to the subject at a dose of about 3.0 mg/kg.

In some embodiments, wherein the anti-PD-L1 antibody or antigen-binding fragment thereof is administered to the subject at a flat dose in the range of about 100 mg to about 1500 mg or at a dose of 2 to 30 mg/kg.

In some embodiments, wherein the anti-PD-L1 antibody or antigen-binding fragment thereof is administered to the subject at a flat dose in the following ranges: 100 to 1400 mg, 100 to 1300 mg, 100 to 1200 mg, 100 to 1100 mg, 100 to 1000 mg, 200 to 1400 mg, 200 to 1300 mg, 200 to 1200 mg, 200 to 1100 mg, 200 to 1000 mg, 300 to 1400 mg, 300 to 1300 mg, 300 to 1200 mg, 300 to 1100 mg, 300 to 1000 mg, 400 to 1400 mg, 400 to 1300 mg, 400 to 1200 mg, 400 to 1100 mg, 400 to 1000 mg, 500 to 1500 mg, 500 to 1400 mg, 500 to 1300 mg, 500 to 1200 mg, 500 to 1100 mg, 500 to 1000 mg, 600 to 1500 mg, 600 to 1400 mg, 600 to 1300 mg, 600 to 1200 mg, 600 to 1100 mg, 600 to 1000 mg, 450 to 550 mg, 550 to 650 mg, 650 to 750 mg, 750 to 850 mg, 550 to 950 mg, 950 to 1050 mg, 1050 to 1150 mg, 1150 to 1250 mg, 1250 to 1350 mg, 1350 to 1450 mg.

In some embodiments, wherein the anti-PD-L1 antibody or antigen-binding fragment thereof is administered to the subject at a flat dose in the following ranges: 600 mg, 900 mg, 1000 mg, 1100 mg, 1200 mg, 1300 mg, 1400 mg, 1500 mg.

In some embodiments, wherein the anti-PD-L1 antibody or antigen-binding fragment thereof is administered to the subject at a flat dose of about 600 mg.

In some embodiments, wherein the anti-PD-L1 antibody or antigen-binding fragment thereof is administered to the subject at a flat dose of about 900 mg.

In some embodiments, wherein the anti-PD-L1 antibody or antigen-binding fragment thereof is administered to the subject at a flat dose of about 1000 mg.

In some embodiments, wherein the anti-PD-L1 antibody or antigen-binding fragment thereof is administered to the subject at a flat dose of about 1100 mg.

In some embodiments, wherein the anti-PD-L1 antibody or antigen-binding fragment thereof is administered to the subject at a flat dose of about 1200 mg.

In some embodiments, wherein the anti-PD-L1 antibody or antigen-binding fragment thereof is administered to the subject at a flat dose of about 1300 mg.

In some embodiments, wherein the anti-PD-L1 antibody or antigen-binding fragment thereof is administered to the subject at a flat dose of about 1400 mg.

In some embodiments, wherein the anti-PD-L1 antibody or antigen-binding fragment thereof is administered to the subject at the following doses: 2.0 mg/kg, 2.4 mg/kg, 2.8 mg/kg, 3.0 mg/kg, 3.5 mg/kg, 4.0 mg/kg, 4.5 mg/kg, 5.0 mg/kg, 5.5 mg/kg, 6.0 mg/kg, 6.5 mg/kg, 7.0 mg/kg, 7.5 mg/kg, 8.0 mg/kg, 8.5 mg/kg, 9.0 mg/kg, 9.5 mg/kg, 10.0 mg/kg, 15 mg/kg, 20 mg/kg, 25 mg/kg, 30 mg/kg.

In some embodiments, wherein the anti-PD-L1 antibody or antigen-binding fragment thereof is administered to the subject at a dose of about 5.0 mg/kg.

In some embodiments, wherein the anti-PD-L1 antibody or antigen-binding fragment thereof is administered to the subject at a dose of about 6.0 mg/kg.

In some embodiments, wherein the anti-PD-L1 antibody or antigen-binding fragment thereof is administered to the subject at a dose of about 7.0 mg/kg.

In some embodiments, wherein the anti-PD-L1 antibody or antigen-binding fragment thereof is administered to the subject at a dose of about 8.0 mg/kg.

In some embodiments, wherein the anti-PD-L1 antibody or antigen-binding fragment thereof is administered to the subject at a dose of about 9.0 mg/kg.

In some embodiments, wherein the anti-PD-L1 antibody or antigen-binding fragment thereof is administered to the subject at a dose of about 10.0 mg/kg.

In some embodiments, wherein the platinum-based drug is administered:
i. to the subject at a dose in the range of about 50 to 150 mg/m²;
   preferably, the platinum-based drug is administered to the subject at the following dose: 50 mg/m², 70 mg/m², 75 mg/m², 80 mg/m², 100 mg/m², 120 mg/m² or 150 mg/m²;
   or
ii. at a dose per administration, calculated as an area under the curve (AUC), selected from 1 to 10 mg/ml/min;
preferably, the platinum-based drug is administered to the subject at the following dose (i.e. injection rate): 3 mg/mL/min, 4 mg/mL/min, 5 mg/mL/min, 6 mg/mL/min or 7 mg/mL/min.

In some embodiments, the platinum-based drug is administered to the subject at the following dose: 50 mg/m², 70 mg/m², 75 mg/m², 80 mg/m², 100 mg/m², 120 mg/m² or 150 mg/m².

In some embodiments, the platinum-based drug is administered to the subject at a dose of about 70 mg/m².

In some embodiments, the platinum-based drug is administered to the subject at a dose of about 75 mg/m².

In some embodiments, the platinum-based drug is administered to the subject at a dose of about 80 mg/m².

In some embodiments, the dose of the carboplatin per administration, calculated as an area under the curve (AUC), is selected from 1 to 10 mg/ml/min, preferably from 1 mg/ml/min, 2 mg/ml/min, 2.5 mg/ml/min, 3 mg/ml/min, 3.75 mg/ml/min, 4 mg/ml/min, 5 mg/ml/min, 6 mg/ml/min, 7 mg/ml/min, 8 mg/ml/min. In some embodiments, the dose of the carboplatin per administration is 3 mg/ml/min, 4 mg/ml/min, 5 mg/ml/min, 6 mg/ml/min, or 7 mg/ml/min.

In some embodiments, the dose of the cisplatin per administration is selected from 50 to 150 mg/m², preferably from 50 mg/m², 70 mg/m², 75 mg/m², 80 mg/m², 100 mg/m², 120 mg/m² and 150 mg/m².

In some embodiments, wherein the anti-B7H3 antibody-drug conjugate, the anti-PD-L1 antibody or antigen-binding fragment thereof, and the platinum-based drug is administered in a dosing cycle of 7 to 42 days. Preferably, the dosing cycle is 21 to 42 days.

In some embodiments, wherein the anti-B7H3 antibody-drug conjugate, the anti-PD-L1 antibody or antigen-binding fragment thereof, and the platinum-based drug is administered in a dosing cycle of 14 days, 21 days, 28 days, 35 days, or 42 days.

In some embodiments, the anti-B7H3 antibody-drug conjugate is administered at a dosing frequency of once daily, once weekly, once every two weeks, once every three weeks, twice every three weeks, once every four weeks, once monthly, once every five weeks, or once every six weeks.

In some embodiments, the anti-B7H3 antibody-drug conjugate is administered at a dosing cycle of one week, two weeks, three weeks, one month, two months, three months, four months, five months, six months, or longer; optionally, the duration of each dosing cycle is the same or different, and the interval between each dosing cycle is the same or different.

In some embodiments, the anti-PD-L1 antibody or antigen-binding fragment thereof is administered at a dosing frequency of once daily, once weekly, once every two weeks, once every three weeks, once every four weeks or once monthly, once every five weeks, once every six weeks.

In some embodiments, the anti-PD-L1 antibody or antigen-binding fragment thereof is administered at a dosing cycle of one week, two weeks, three weeks, one month, two months, three months, four months, five months, six months, or longer; optionally, the duration of each dosing cycle is the same or different, and the interval between each dosing cycle is the same or different.

In some embodiments, the platinum-based drug (for example, carboplatin or cisplatin) is administered at a dosing frequency of once daily, once weekly, once every two weeks, once every three weeks, once every four weeks or once monthly, once every five weeks, once every six weeks.

In some embodiments, the platinum-based drug (for example, carboplatin or cisplatin) is administered at a dosing cycle of one week, two weeks, three weeks, one month, two months, three months, four months, five months, six months, or longer; optionally, the duration of each dosing cycle is the same or different, and the interval between each dosing cycle is the same or different.

In some embodiments, the anti-B7H3 antibody-drug conjugate and the additional therapeutic agent are administered simultaneously.

In some embodiments, the anti-B7H3 antibody-drug conjugate and the additional therapeutic agent are administered separately.

In some embodiments, the anti-B7H3 antibody-drug conjugate and the additional therapeutic agent are administered sequentially.

In some embodiments, the anti-B7H3 antibody-drug conjugate is administered first, followed by the administration of the additional therapeutic agent.

In some embodiments, the cancer is selected from a solid tumour.

In some embodiments, the cancer is selected from nasopharyngeal carcinoma, colorectal cancer, non-small cell lung cancer, small cell lung cancer, oesophageal squamous cell carcinoma, prostate cancer, head and neck squamous cell carcinoma.

In some embodiments, the cancer is selected from nasopharyngeal carcinoma, non-small cell lung cancer, small cell lung cancer.

In some embodiments, the cancer is selected from non-small cell lung cancer.

In some embodiments, the non-small cell lung cancer (NSCLC) is selected from squamous NSCLC, adenocarcinoma NSCLC, pulmonary lymphoepithelioma-like carcinoma, large cell lung cancer.

In some embodiments, the non-small cell lung cancer (NSCLC) is selected from those positive for driver genes or negative for driver genes.

In some embodiments, the nasopharyngeal carcinoma is selected from keratinising squamous cell carcinoma of the nasopharynx, non-keratinising nasopharyngeal carcinoma (including differentiated or undifferentiated types), basaloid squamous cell carcinoma of the nasopharynx, nasopharyngeal carcinoma in situ, invasive nasopharyngeal carcinoma (including, but not limited to, squamous cell carcinoma, adenocarcinoma, microinvasive carcinoma, vesicular nucleus carcinoma or undifferentiated nasopharyngeal carcinoma).

In some embodiments, the non-keratinising nasopharyngeal carcinoma includes differentiated or undifferentiated types.

In some embodiments, the invasive nasopharyngeal carcinoma includes, but is not limited to, squamous cell carcinoma, adenocarcinoma, microinvasive carcinoma, vesicular nucleus carcinoma or undifferentiated nasopharyngeal carcinoma.

In some embodiments, the subject is a patient with locally recurrent or metastatic nasopharyngeal carcinoma who has previously failed treatment with a platinum-containing regimen.

In some embodiments, the subject is a patient with locally recurrent or metastatic nasopharyngeal carcinoma who has not received systemic treatment.

In some embodiments, the subject is a patient with small cell lung cancer who has not received systemic treatment.

In some embodiments, the subject is a patient with locally advanced or metastatic non-small cell lung cancer who has previously failed treatment with a platinum-containing regimen and who is negative for epidermal growth factor receptor (EGFR) sensitising mutations, negative for anaplastic lymphoma kinase (ALK), and/or negative for c-ros proto-oncogene (ROS1).

In some embodiments, the subject is a patient with locally advanced or metastatic non-small cell lung cancer who has previously failed treatment with a platinum-containing regimen and who is negative for epidermal growth factor receptor (EGFR) sensitising mutations and negative for anaplastic lymphoma kinase (ALK)/c-ros proto-oncogene (ROS1).

In some embodiments, the subject is a patient with locally advanced or metastatic non-small cell lung cancer who has not received systemic treatment and who is negative for EGFR sensitising mutations, negative for ALK and/or negative for c-ros proto-oncogene (ROS1).

In some embodiments, the subject is a patient with locally advanced or metastatic non-small cell lung cancer who has not received systemic treatment and who is negative for EGFR sensitising mutations and negative for ALK/c-ros proto-oncogene (ROS1).

In some embodiments, the anti-B7H3 antibody-drug conjugate and the additional therapeutic agent are present in the form of unit dosage.

In some embodiments, the anti-B7H3 antibody-drug conjugate and the additional therapeutic agent are present in the same dosage unit.

In some embodiments, the anti-B7H3 antibody-drug conjugate and the additional therapeutic agent are present in different dosage units.

In some embodiments, the route of dosing may be oral dosing, parenteral dosing, transdermal dosing, and the parenteral dosing includes, but is not limited to, intravenous injection, subcutaneous injection, intramuscular injection.

In some embodiments, the route of administration of the anti-B7H3 antibody-drug conjugate and the additional therapeutic agent is intravenous injection.

In some embodiments, the anti-B7H3 antibody-drug conjugate, or a pharmaceutically acceptable salt, stereoisomer or metabolite thereof or a solvate of any of the foregoing is administered in a manner of injection; and prior to injection, the anti-B7H3 antibody-drug conjugate, or a pharmaceutically acceptable salt, stereoisomer or metabolite thereof or a solvate of any of the foregoing needs to be formulated into an injectable form, the preferred injection form is an injection solution or lyophilised powder, comprising the anti-B7H3 antibody-drug conjugate, a pharmaceutically acceptable salt, stereoisomer or metabolite thereof, or a solvate of any of the foregoing, and optionally, a buffering agent, a stabiliser, a pH adjusting agent and a surfactant, wherein the buffering agent may be selected from one or more of acetate, citrate, succinate and phosphate; the stabiliser may be selected from a sugar or an amino acid, preferably a disaccharide, such as sucrose, lactose, trehalose or maltose; the pH adjusting agent may be selected from one or more of sodium hydroxide, lithium hydroxide and potassium hydroxide; the surfactant may be selected from polyoxyethylene hydrogenated castor oil, glycerol fatty acid ester and polyoxyethylene sorbitan fatty acid ester, and preferably the polyoxyethylene sorbitan fatty acid ester is polysorbate 20, 40, 60 or 80, most preferably polysorbate 20.

In some embodiments, wherein one or more therapeutic effects in the subject are improved relative to the baseline following administration of the anti-B7H3 antibody-drug conjugate and the anti-PD-L1 antibody or antigen-binding fragment thereof, wherein the one or more therapeutic effects are selected from size of tumour derived from the cancer, objective response rate (ORR), depth of response (DpR), disease control rate (DCR), duration of response (DoR), time to response (TTR), progression-free survival (PFS), and overall survival (OS).

In some embodiments, the size of tumour derived from the cancer of the subject is reduced by at least about 10%, at least about 15%, at least about 20%, at least about 25%, at least about 30%, at least about 35%, at least about 40%, at least about 45%, at least about 50%, at least about 60%, at least about 70%, or at least about 80%, relative to the size of tumour prior to administration of the anti-B7H3 antibody-drug conjugate and the anti-PD-L1 antibody or antigen-binding fragment thereof.

In a third aspect, the present disclosure further provides a pharmaceutical composition, comprising an anti-B7H3 antibody-drug conjugate and an additional therapeutic agent, wherein the additional therapeutic agent is selected from one or both of an anti-PD-L1 antibody or antigen-binding fragment thereof and a chemotherapy drug, and the anti-B7H3 antibody-drug conjugate, the anti-PD-L1 antibody or antigen-binding fragment thereof, and the chemotherapy drug are as defined herein.

In some embodiments, the pharmaceutical composition is a pharmaceutical composition for treating cancer, and the cancer is as defined above.

In some embodiments, the pharmaceutical composition further comprises one or more pharmaceutically acceptable excipients, diluents, or carriers.

In some embodiments, the pharmaceutical composition comprises an anti-B7H3 antibody-drug conjugate and an additional therapeutic agent, wherein the additional therapeutic agent is selected from: i. atezolizumab; or, ii. atezolizumab and a platinum-based drug;
the anti-B7H3 antibody-drug conjugate is an antibody-drug conjugate having the structure shown in formula I, a pharmaceutically acceptable salt thereof: 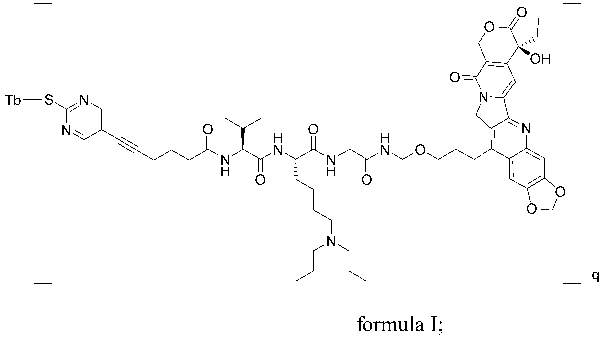
wherein S is a sulphur atom on Tb; Tb is an anti-B7H3 antibody or antigen-binding fragment thereof; q is selected from any numerical value ranging from 0.1 to 16.0; preferably, q is selected from 1, 2, 3, 4, 5, 6, 7, 8, 9, 10; more preferably, q is 2, 4, 6 or 8; still more preferably, q is 8;
the anti-B7H3 antibody or antigen-binding fragment thereof comprises a heavy chain variable region and a light chain variable region, wherein:
   the heavy chain variable region (VH) comprises:
      (i) CDR-H1 comprising the amino acid sequence set forth in SEQ ID NO: 1;
      (ii) CDR-H2 comprising the amino acid sequence set forth in SEQ ID NO: 2; and
      (iii) CDR-H3 comprising the amino acid sequence set forth in SEQ ID NO: 3; and
   the light chain variable region (VL) comprises:
      (i) CDR-L1 comprising the amino acid sequence set forth in SEQ ID NO: 4;
      (ii) CDR-L2 comprising the amino acid sequence set forth in SEQ ID NO: 5; and
      (iii) CDR-L3 comprising the amino acid sequence set forth in SEQ ID NO: 6;
   wherein the CDRs are determined in accordance with the Kabat definition scheme.

In a fourth aspect, the present disclosure further provides a kit, comprising an anti-B7H3 antibody-drug conjugate and an additional therapeutic agent, wherein the additional therapeutic agent is selected from one or both of an anti-PD-L1 antibody or antigen-binding fragment thereof and a chemotherapy drug, and the anti-B7H3 antibody-drug conjugate, the anti-PD-L1 antibody or antigen-binding fragment thereof, and the chemotherapy drug are as defined herein.

In some embodiments, the kit is a kit for treating cancer, and the cancer is as defined above.

In some embodiments, the kit comprises an anti-B7H3 antibody-drug conjugate and an additional therapeutic agent, wherein the additional therapeutic agent is selected from: i. atezolizumab; or, ii. atezolizumab and a platinum-based drug;
the anti-B7H3 antibody-drug conjugate is an antibody-drug conjugate having the structure shown in formula I, a pharmaceutically acceptable salt thereof: 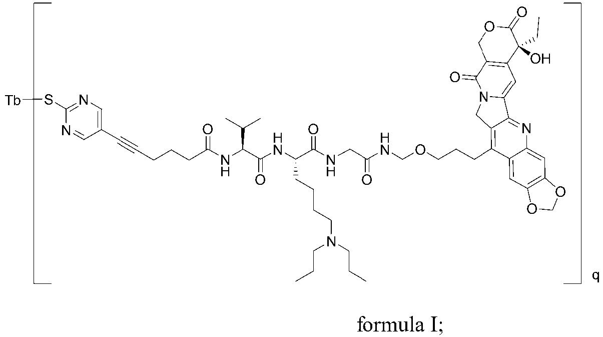
wherein S is a sulphur atom on Tb; Tb is an anti-B7H3 antibody or antigen-binding fragment thereof; q is selected from any numerical value ranging from 0.1 to 16.0; preferably, q is selected from 1, 2, 3, 4, 5, 6, 7, 8, 9, 10; more preferably, q is 2, 4, 6 or 8; still more preferably, q is 8;
the anti-B7H3 antibody or antigen-binding fragment thereof comprises a heavy chain variable region and a light chain variable region, wherein:
   the heavy chain variable region (VH) comprises:
      (i) CDR-H1 comprising the amino acid sequence set forth in SEQ ID NO: 1;
      (ii) CDR-H2 comprising the amino acid sequence set forth in SEQ ID NO: 2; and
      (iii) CDR-H3 comprising the amino acid sequence set forth in SEQ ID NO: 3; and
   the light chain variable region (VL) comprises:
      (i) CDR-L1 comprising the amino acid sequence set forth in SEQ ID NO: 4;
      (ii) CDR-L2 comprising the amino acid sequence set forth in SEQ ID NO: 5; and
      (iii) CDR-L3 comprising the amino acid sequence set forth in SEQ ID NO: 6;
   wherein the CDRs are determined in accordance with the Kabat definition scheme.

In some embodiments, the kit comprises a container A and a container B, wherein the container A comprises an anti-B7H3 antibody-drug conjugate as an active ingredient, and the container B comprises the additional therapeutic agent as an active ingredient.

In a fifth aspect, the present disclosure further provides use of the kit according to the fourth aspect in the preparation of a drug for treating cancer.

In a sixth aspect, the present disclosure provides a pharmaceutical combination, comprising an anti-B7H3 antibody-drug conjugate and an additional therapeutic agent, wherein the additional therapeutic agent is selected from one or both of an anti-PD-L1 antibody or antigen-binding fragment thereof and a chemotherapy drug, and the anti-B7H3 antibody-drug conjugate, the anti-PD-L1 antibody or antigen-binding fragment thereof, and the chemotherapy drug are as defined above. In a seventh aspect, the present disclosure provides use of a combination of an anti-B7H3 antibody-drug conjugate and an additional therapeutic agent in the preparation of a drug for treating cancer.

In some embodiments, there is further provided use of a combination of the anti-B7H3 antibody-drug conjugate and the anti-PD-L1 antibody or antigen-binding fragment thereof as described above in the preparation of a drug for treating cancer.

In some embodiments, there is also provided use of a combination of one or both of the anti-B7H3 antibody-drug conjugate and the anti-PD-L1 antibody or antigen-binding fragment thereof as well as a chemotherapy drug in the preparation of a drug for treating cancer.

In one aspect, the present disclosure also provides the aforementioned kit or pharmaceutical combination for treating cancer.

In a further aspect, there is also provided use of an anti-B7H3 antibody-drug conjugate in the preparation of a drug for treating cancer, the anti-B7H3 antibody-drug conjugate is administered in combination with an additional therapeutic agent, and the anti-B7H3 antibody-drug conjugate is as defined above.

In a further aspect, there is also provided an anti-B7H3 antibody-drug conjugate for treating cancer, and the anti-B7H3 antibody-drug conjugate is as defined above and is administered in combination with an additional therapeutic agent.

In some embodiments, there is also provided an anti-B7H3 antibody-drug conjugate for use in combination with atezolizumab in treating cancer, wherein the anti-B7H3 antibody-drug conjugate is an antibody-drug conjugate having the structure shown in formula I, a pharmaceutically acceptable salt, stereoisomer or metabolite thereof, or a solvate of any of the foregoing: 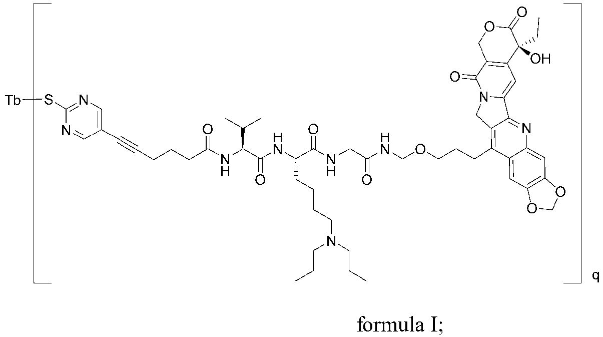
wherein S is a sulphur atom on Tb; Tb is an anti-B7H3 antibody or antigen-binding fragment thereof; q is selected from any numerical value ranging from 0.1 to 16.0; preferably, q is selected from 1, 2, 3, 4, 5, 6, 7, 8, 9, 10; more preferably, q is 2, 4, 6 or 8; still more preferably, q is 8;
the anti-B7H3 antibody or antigen-binding fragment thereof comprises a heavy chain variable region and a light chain variable region, wherein:
   the heavy chain variable region (VH) comprises:
      (i) CDR-H1 comprising the amino acid sequence set forth in SEQ ID NO: 1;
      (ii) CDR-H2 comprising the amino acid sequence set forth in SEQ ID NO: 2; and
      (iii) CDR-H3 comprising the amino acid sequence set forth in SEQ ID NO: 3; and
   the light chain variable region (VL) comprises:
      (i) CDR-L1 comprising the amino acid sequence set forth in SEQ ID NO: 4;
      (ii) CDR-L2 comprising the amino acid sequence set forth in SEQ ID NO: 5; and
      (iii) CDR-L3 comprising the amino acid sequence set forth in SEQ ID NO: 6;
   wherein the CDRs are determined in accordance with the Kabat definition scheme.

In some embodiments, there is also provided an anti-B7H3 antibody-drug conjugate for use in combination with atezolizumab and a platinum-based drug in treating cancer, wherein the anti-B7H3 antibody-drug conjugate is an antibody-drug conjugate having the structure shown in formula I, a pharmaceutically acceptable salt, stereoisomer or metabolite thereof, or a solvate of any of the foregoing: 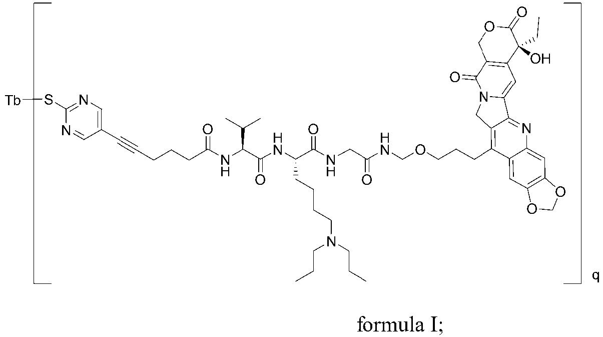
wherein S is a sulphur atom on Tb; Tb is an anti-B7H3 antibody or antigen-binding fragment thereof; q is selected from any numerical value ranging from 0.1 to 16.0; preferably, q is selected from 1, 2, 3, 4, 5, 6, 7, 8, 9, 10; more preferably, q is 2, 4, 6 or 8; still more preferably, q is 8;
the anti-B7H3 antibody or antigen-binding fragment thereof comprises a heavy chain variable region and a light chain variable region, wherein:
   the heavy chain variable region (VH) comprises:
      (i) CDR-H1 comprising the amino acid sequence set forth in SEQ ID NO: 1;
      (ii) CDR-H2 comprising the amino acid sequence set forth in SEQ ID NO: 2; and
      (iii) CDR-H3 comprising the amino acid sequence set forth in SEQ ID NO: 3; and
   the light chain variable region (VL) comprises:
      (i) CDR-L1 comprising the amino acid sequence set forth in SEQ ID NO: 4;
      (ii) CDR-L2 comprising the amino acid sequence set forth in SEQ ID NO: 5; and
      (iii) CDR-L3 comprising the amino acid sequence set forth in SEQ ID NO: 6;
   wherein the CDRs are determined in accordance with the Kabat definition scheme.

In some embodiments, there is also provided a PD-L1 antibody for use in combination with an anti-B7H3 antibody-drug conjugate in treating cancer, wherein the PD-L1 antibody is atezolizumab, and the anti-B7H3 antibody-drug conjugate is an antibody-drug conjugate having the structure shown in formula I, a pharmaceutically acceptable salt, stereoisomer or metabolite thereof, or a solvate of any of the foregoing: 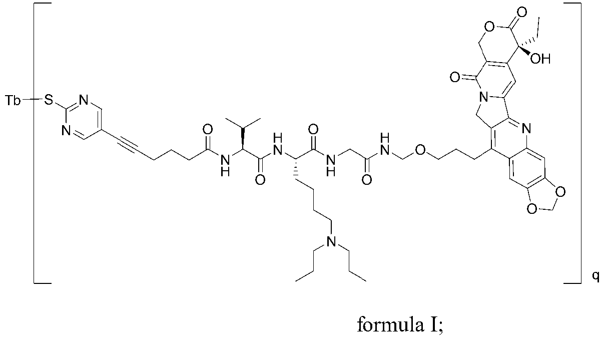
wherein S is a sulphur atom on Tb; Tb is an anti-B7H3 antibody or antigen-binding fragment thereof; q is selected from any numerical value ranging from 0.1 to 16.0; preferably, q is selected from 1, 2, 3, 4, 5, 6, 7, 8, 9, 10; more preferably, q is 2, 4, 6 or 8; still more preferably, q is 8;
the anti-B7H3 antibody or antigen-binding fragment thereof comprises a heavy chain variable region and a light chain variable region, wherein:
   the heavy chain variable region (VH) comprises:
      (i) CDR-H1 comprising the amino acid sequence of SEQ ID NO: 1;
      (ii) CDR-H2 comprising the amino acid sequence of SEQ ID NO: 2; and
      (iii) CDR-H3 comprising the amino acid sequence of SEQ ID NO: 3; and
   the light chain variable region (VL) comprises:
      (i) CDR-L1 comprising the amino acid sequence of SEQ ID NO: 4;
      (ii) CDR-L2 comprising the amino acid sequence of SEQ ID NO: 5; and
      (iii) CDR-L3 comprising the amino acid sequence of SEQ ID NO: 6;
   wherein the CDRs are determined in accordance with the Kabat definition scheme.

In some embodiments, there is also provided a PD-L1 antibody for use in combination with an anti-B7H3 antibody-drug conjugate and a platinum-based drug in treating cancer, wherein the PD-L1 antibody is atezolizumab, and the anti-B7H3 antibody-drug conjugate is an antibody-drug conjugate having the structure shown in formula I, a pharmaceutically acceptable salt, stereoisomer or metabolite thereof, or a solvate of any of the foregoing: 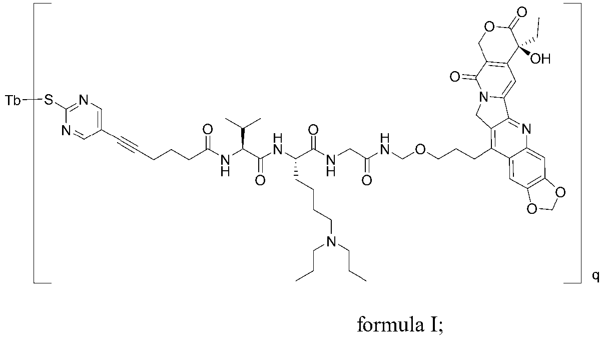
wherein S is a sulphur atom on Tb; Tb is an anti-B7H3 antibody or antigen-binding fragment thereof; q is selected from any numerical value ranging from 0.1 to 16.0; preferably, q is selected from 1, 2, 3, 4, 5, 6, 7, 8, 9, 10; more preferably, q is 2, 4, 6 or 8; still more preferably, q is 8;
the anti-B7H3 antibody or antigen-binding fragment thereof comprises a heavy chain variable region and a light chain variable region, wherein:
   the heavy chain variable region (VH) comprises:
      (i) CDR-H1 comprising the amino acid sequence set forth in SEQ ID NO: 1;
      (ii) CDR-H2 comprising the amino acid sequence set forth in SEQ ID NO: 2; and
      (iii) CDR-H3 comprising the amino acid sequence set forth in SEQ ID NO: 3; and
   the light chain variable region (VL) comprises:
      (i) CDR-L1 comprising the amino acid sequence set forth in SEQ ID NO: 4;
      (ii) CDR-L2 comprising the amino acid sequence set forth in SEQ ID NO: 5; and
      (iii) CDR-L3 comprising the amino acid sequence set forth in SEQ ID NO: 6;
   wherein the CDRs are determined in accordance with the Kabat definition scheme.

In some embodiments, there is also provided a combination of an anti-B7H3 antibody-drug conjugate and atezolizumab for use in treating cancer, wherein the anti-B7H3 antibody-drug conjugate is an antibody-drug conjugate having the structure shown in formula I, a pharmaceutically acceptable salt, stereoisomer or metabolite thereof, or a solvate of any of the foregoing: 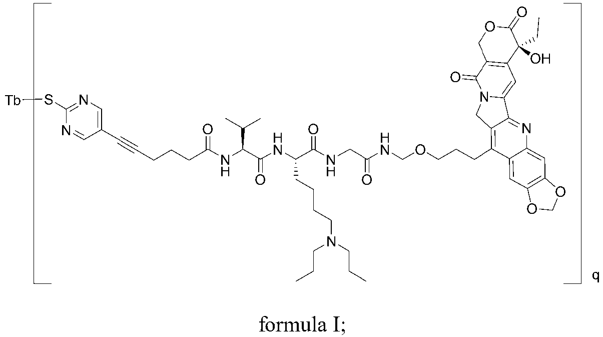
wherein S is a sulphur atom on Tb; Tb is an anti-B7H3 antibody or antigen-binding fragment thereof; q is selected from any numerical value ranging from 0.1 to 16.0; preferably, q is selected from 1, 2, 3, 4, 5, 6, 7, 8, 9, 10; more preferably, q is 2, 4, 6 or 8; still more preferably, q is 8;
the anti-B7H3 antibody or antigen-binding fragment thereof comprises a heavy chain variable region and a light chain variable region, wherein:
   the heavy chain variable region (VH) comprises:
      (i) CDR-H1 comprising the amino acid sequence set forth in SEQ ID NO: 1;
      (ii) CDR-H2 comprising the amino acid sequence set forth in SEQ ID NO: 2; and
      (iii) CDR-H3 comprising the amino acid sequence set forth in SEQ ID NO: 3; and
   the light chain variable region (VL) comprises:
      (i) CDR-L1 comprising the amino acid sequence set forth in SEQ ID NO: 4;
      (ii) CDR-L2 comprising the amino acid sequence set forth in SEQ ID NO: 5; and
      (iii) CDR-L3 comprising the amino acid sequence set forth in SEQ ID NO: 6;
   wherein the CDRs are determined in accordance with the Kabat definition scheme.

In some embodiments, treatment with a combination of an anti-B7H3 antibody-drug conjugate and an immune checkpoint inhibitor has a significant inhibitory effect on tumours. In a mouse tumour-bearing model, compared with monotherapy, combined administration of the anti-B7H3 antibody-drug conjugate and an anti-PD-L1 antibody can synergistically inhibit tumour growth and produce a significant antitumour effect.

In some embodiments, the present disclosure further experimentally verifies the inhibitory effect of the combination of the anti-B7H3 antibody-drug conjugate and the anti-PD-L1 antibody and/or the chemotherapy drug on tumours. Not limited to verification methods of animal tumour models, in vitro cell models, human clinical trials, and the like are also included; tumour types are not limited to colorectal cancer, but may also be other tumours, including but not limited to nasopharyngeal carcinoma, non-small cell lung cancer, small cell lung cancer, oesophageal squamous cell carcinoma, prostate cancer, head and neck squamous cell carcinoma, and the like.

### DETAILED DESCRIPTION OF THE INVENTION

Unless defined otherwise, all terms used herein have the same meanings as commonly understood by those of ordinary skill in the art. For related definitions and terms, reference may be made, for example, to Current Protocols in Molecular Biology (Ausubel). Meanwhile, for a better understanding of the present disclosure, definitions and explanations of relevant terms are provided below.

All documents mentioned herein are incorporated herein by reference in their entirety.

The B7H3 antibodies with native sequences herein may be isolated from nature, or may be prepared by recombinant DNA techniques, chemical synthesis methods, or combinations thereof.

As used herein, the term "antibody" is used in its broadest sense and comprises intact monoclonal antibodies, polyclonal antibodies and multispecific antibodies formed from at least two intact antibodies (e.g., bispecific antibodies), so long as they have desired biological activity.

As used herein, the term "monoclonal antibody" refers to an antibody from a population of substantially homogeneous antibodies, i.e. all antibodies constituting the population are identical except for a small number of natural mutations that may be present. Monoclonal antibodies have high specificity to one determinant (epitope) of an antigen, whereas polyclonal antibodies opposite thereto comprise different antibodies directed against different determinants (epitopes). In addition to specificity, an advantage of monoclonal antibodies also lies in the fact that they may be synthesised free from contamination by other antibodies. The modifier "monoclonal" here represents that the antibody is characterized by being from a substantially homogeneous population of antibodies, and is not to be construed as requiring preparation by any particular method.

As used herein, monoclonal antibodies also specifically comprise chimeric antibodies, that is, part of the heavy chain and/or light chain is identical or homologous to some, certain class of or certain subclass of antibodies, while the remainder is identical or homologous to some other, another class of or another subclass of antibodies, as long as they have the desired biological activity (see, for example, US4,816,567; and Morrison et al., 1984, PNAS, 81: 6851-6855). Chimeric antibodies that may be used in the present invention comprise primatised antibodies, which comprise variable region antigen-binding sequences from non-human primates (e.g., Old World monkeys, orangutans, and the like) and human constant region sequences.

As used herein, "a plurality" comprises more than one choice, for example, two, three, four, or more choices. In a particular embodiment, "a plurality" represents at least two.

The term "antibody fragment" refers to a portion of an antibody, preferably the antigen-binding region or variable region. Examples of antibody fragments comprise Fab, Fab', F(ab')₂ and Fv fragments; diabodies; linear antibodies; and single-chain antibody molecules.

In the present disclosure, the term "drug" refers to a substance that inhibits or prevents the function of cells and/or causes cell death or destruction.

The antibody-drug conjugate of the present invention may be in the form of a pharmaceutically acceptable salt, or in the form of a stereoisomer, or in the form of a metabolite, or in the form of a solvate, and the salt, stereoisomer or metabolite may also be in the form of a solvate.

The term "pharmaceutically acceptable salt" refers to salts that retain the bio-availability and properties of a compound, and they are biologically or otherwise desirable for use as a drug. In many cases, the antibody-drug conjugate of the present invention may form acid addition salts and/or base addition salts by virtue of amino groups and/or carboxyl groups or similar groups present therein.

The pharmaceutically acceptable acid addition salts may be salts formed with inorganic acids or organic acids. The inorganic acids comprise, for example, hydrochloric acid, hydrobromic acid, sulphuric acid, nitric acid and phosphoric acid, and the like. The organic acids comprise, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulphonic acid, ethanesulphonic acid, p-toluenesulphonic acid and salicylic acid, and the like.

Pharmaceutically acceptable base addition salts may be salts formed with inorganic bases or organic bases. The salts formed with inorganic bases comprise, for example, sodium salts, potassium salts, lithium salts, ammonium salts, calcium salts, magnesium salts, iron salts, zinc salts, copper salts, manganese salts and aluminium salts, and the like, particularly preferably, ammonium salts, potassium salts, sodium salts, calcium salts and magnesium salts. The organic bases comprise, for example, primary amines, secondary amines and tertiary amines, substituted amines (including naturally occurring substituted amines), cyclic amines, basic ion exchange resins, and the like. Specific examples of organic bases are isopropylamine, trimethylamine, diethylamine, N-ethylethylamine, tripropylamine and ethanolamine.

Pharmaceutically acceptable salts can be obtained using standard procedures well known in the art, for example, by reacting a sufficient quantity of basic compounds with a suitable acid which provides pharmaceutically acceptable anions.

The term "stereoisomer" represents an isomer formed due to at least one asymmetric centre. In a compound having one or more asymmetric centres, it can produce racemates, racemic mixtures, single enantiomers, diastereomeric mixtures and individual diastereomers. Particular individual molecules may also be present as geometric isomers (cis/trans). Unless otherwise indicated, when the stereochemistry of the disclosed compound is not explicitly specified in the name or structure of the disclosed compound and the disclosed compound has one or more asymmetric centres, it should be understood to represent all possible stereoisomers of the compound.

The term "solvate" refers to a solvate formed by association of one or more solvent molecules with an antibody-drug conjugate of any formula (I) or a pharmaceutically acceptable salt or isomer thereof. The term solvate includes hydrates (for example, hemihydrate, monohydrate, dihydrate, trihydrate, tetrahydrate and similar hydrates).

Also included within the scope of the present disclosure are metabolites of the compounds of the present invention, that is, substances formed in vivo when the compounds of the present invention are administered. Such products can result from, for example, oxidation, reduction, hydrolysis, amidation, deamidation, esterification, delipidation, enzymatic cleavage, and the like, of the administered compound. Accordingly, the present invention includes metabolites of the compounds of the present invention, including compounds produced by a method of contacting a compound of the present invention with a mammal for a time sufficient to produce a metabolite thereof.

As used herein, the term "treatment" refers to a method implemented in order to obtain a beneficial or desired clinical result. For purposes of the present invention, beneficial or desired clinical results include, but are not limited to, alleviating symptoms, reducing disease lesions, stabilising (that is, no longer worsening) the state of disease, delaying or slowing the progression of disease, ameliorating or alleviating the state of disease, and relieving symptoms (whether partially or completely), whether detectable or undetectable. Furthermore, "treatment" may also refer to prolongation of survival relative to expected survival (if not receiving treatment).

As used herein, the term "progression of a disease" or "disease progression" refers to an increase of at least 20% in the sum of diameters of target lesions, taking as reference the smallest sum of diameters recorded for all target lesions throughout the study (including the baseline sum if that is the smallest value), together with an absolute increase of at least 5 mm in the sum of diameters; alternatively, disease progression may be indicated by the appearance of one or more new lesions.

As used herein, the term "therapeutically effective amount" or "effective amount" refers to an amount of an anti-B7H3 antibody-drug conjugate (anti-B7H3-ADC) and/or an anti-PD-L1 antibody or antigen-binding fragment thereof and/or a chemotherapy drug that is effective, when administered to a cell, tissue or subject being treated, in preventing or slowing the disease or condition to be treated. A therapeutically effective dose further refers to an amount of an anti-B7H3 antibody-drug conjugate (anti-B7H3-ADC) and/or a PD-L1 antibody or antigen-binding fragment thereof and/or a chemotherapy drug sufficient to produce alleviation of symptoms, for example including treatment, cure or alleviation of the relevant medical condition, or improvement in the rate of treatment, cure, or alleviation of the condition. The effective amount for a specific subject being treated may vary depending on a variety of factors, such as the disease to be treated, the overall health status of the patient, the method, route and dose of administration, and the severity of side effects. An effective amount may be the maximum dose or dosing regimen that avoids significant side effects or toxic effects. A therapeutically effective amount will typically alleviate symptoms by at least 10%, typically by at least 20%, by at least about 30%, by at least 40%, or by at least 50%.

As used herein, the terms "individual", "subject" or "patient" refer to any animal that is as the target of treatment, observation or experimentation, including (but not limited to) non-human primates, rodents, and the like. Typically, when referring to a human individual, the terms "subject" and "patient" are used interchangeably herein. The mammal may be one or more selected from humans, bovines (for example, cattle), pig-like animals (for example, pigs), sheep-like animals (for example, sheep), goat-like animals (for example, goats), equines (for example, horses), canines (such as domestic dogs), felines (such as domestic cats), lagomorphs (such as rabbits), rodents (such as rats or mice), and the like. In particular embodiments, the subject is a human.

As used herein, the terms "complete response (CR)", "partial response (PR)", "stable disease (SD)", "progressive disease (PD)", "objective response rate (ORR)", "disease control rate (DCR)" are defined according to the following methods:
When the disease is a solid tumour, the efficacy for solid tumours may be evaluated as "complete response (CR)", "partial response (PR)", "stable disease (SD)", "progressive disease (PD)", "objective response rate (ORR)", "disease control rate (DCR)" according to the following criteria (see New response evaluation criteria in solid tumours: Revised RECIST guideline (version 1.1), E.A. Eisenhauer et al., "EUROPEAN JOURNAL OF CANCER", 45 (2009), pp. 228-247). The evaluation of target lesions is specifically as follows:
Complete response (CR): all target lesions disappear, the short diameters of all pathological lymph nodes (including target nodes and non-target nodes) must be reduced to <10 mm, and there are no new lesions.

Partial response (PR): the sum of the diameters of target lesions (the short diameters are taken for lymph nodes) is reduced by at least 30% compared with the baseline level. There are no obvious progression of non-target lesions and no new lesions.

Stable disease (SD): the degree of reduction in target lesions does not reach PR, the degree of increase also does not reach the PD level, the response therefor lies between the PR and the PD level, and the smallest value of the sum of the diameters during the study may be used as a reference.

Progressive disease (PD): taking the smallest value of the sum of the diameters of all measured target lesions as a reference during the experimental study process, the sum of the diameters is relatively increased by at least 20% (the baseline value is taken as a reference if the baseline measured value is the smallest); in addition, the absolute value of the sum of the diameters of the measured target lesions must increase by at least 5 mm (the presence of one or more new lesions is also considered as the progressive disease).

Objective response rate (ORR): it refers to the proportion of patients whose tumours have shrunk to a defined extent, including cases of CR and PR. Subjects must have measurable tumour lesions at baseline, and the efficacy evaluation criteria is according to RECIST 1.1.

Disease control rate (DCR): the percentage of patients who achieved complete response (CR) and partial response (PR) as well as stable disease (SD) among the total number of participants in the analysis.

PFS and OS can be measured according to the criteria set according to the National Cancer Institute and the U.S. Food and Drug Administration with respect to new drug approval. See Johnson et al., J. Clin. Oncol. 21(7):1404-1411 (2003).

Progression-free survival (PFS) refers to the time from enrollment to disease progression or death. PFS is generally measured using the Kaplan-Meier method and the Response Evaluation Criteria in Solid Tumours (RECIST) 1.1 criteria. Generally, progression-free survival refers to the state where the patient remains alive and the cancer has not worsened.

"Overall survival" (OS) refers to the time from enrollment of a patient to death or to the date on which the patient is last known to be alive. OS comprises an extension of the expected life span compared with untreated or non-treated individuals or patients. Overall survival refers to the state where the patient remains alive for a specified period of time, for example one year, five years, etc. from diagnosis or treatment. In a patient population, overall survival is measured by the median overall survival (mOS).

Depth of response (DpR): the depth of response refers to the degree of reduction in tumour volume or disease burden during treatment, and specifically refers to the percentage of maximum tumour shrinkage achieved during treatment compared to the baseline; if CR occurs, DpR is 100. It is usually measured on the basis of imageology or clinical assessment.

Duration of response (DoR): the duration of response refers to the duration of the tumour response state after treatment, and specifically refers to the time from the time when subjects who have achieved a confirmed complete response (CR) or a confirmed partial response (PR) achieve the confirmed complete response (CR) or the confirmed partial response (PR) for the first time to the time of disease progression for the first time.

Time to response (TTR): the time to response refers to the time from the start of treatment to the time when a patient achieves a response (CR or PR) state for the first time.

### BRIEF DESCRIPTION OF THE DRAWINGS

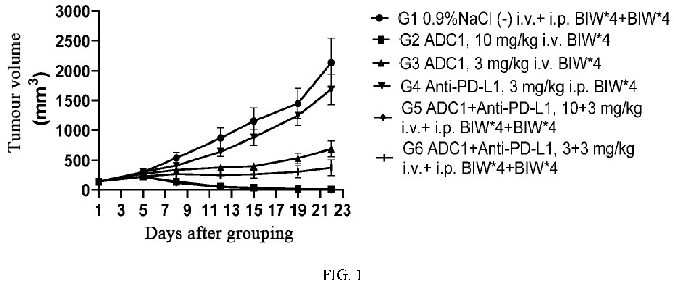

Figure 1: Testing situations of pharmaceutical effects of antibody-drug conjugate (ADC1) in combination with anti-PD-L1 drug on colorectal cancer.

### DETAILED DESCRIPTION OF EMBODIMENTS

### Sequence information

The sequence information involved in the present application is described in the table below:

| SEQ ID NO | Name | Sequence |
|---|---|---|
| 1 | CDR-H1 | SYAMN |
| 2 | CDR-H2 | GISGSGGSTYYADSVQG |
| 3 | CDR-H3 | ARSGYDYFFDY |
| 4 | CDR-L1 | RASQRISSSFLA |
| 5 | CDR-L2 | GASSRAT |
| 6 | CDR-L3 | QQYSNSPLT |
| 7 | Heavy chain variable region (VH) | EVQLLESGGGLVQPGGSLRLSCAASGFTFSSYAMNWVRQAPGKGLEWV SGISGSGGSTYYADSVQGRFTISRDNSKNTLYLQMNSLRAEDTAVYYCAK ARSGYDYFFDYWGQGTLVTVSS |
| 8 | Light chain variable region (VL) | EIVLTQSPGTLSLSPGERATLSCRASQRISSSFLAWYQQKPGQAPSLLIYGA SSRATGIPDRFSGSGSGTDFTLTISSLEPEDFAVFYCQQYSNSPLTFGGGTK VEIK |
| 9 | Heavy chain (HC) | EVQLLESGGGLVQPGGSLRLSCAASGFTFSSYAMNWVRQAPGKGLEWV SGISGSGGSTYYADSVQGRFTISRDNSKNTLYLQMNSLRAEDTAVYYCAK ARSGYDYFFDYWGQGTLVTVSSASTKGPSVFPLAPSSKSTSGGTAALGCL VKDYFPEPVTVSWNSGALTSGVHTFPAVLQSSGLYSLSSVVTVPSSSLGT QTYICNVNHKPSNTKVDKKVEPKSCDKTHTCPPCPAPELLGGPSVFLFPP KPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNYK TTPPVLDSDGSFFLYSKLTVDKSRWQQGNVFSCSVMHEALHNHYTQKSL SLSPGK |
| 10 | Light chain (LC) | EIVLTQSPGTLSLSPGERATLSCRASQRISSSFLAWYQQKPGQAPSLLIYGA SSRATGIPDRFSGSGSGTDFTLTISSLEPEDFAVFYCQQYSNSPLTFGGGTK VEIKRTVAAPSVFIFPPSDEQLKSGTASVVCLLNNFYPREAKVQWKVDNA LQSGNSQESVTEQDSKDSTYSLSSTLTLSKADYEKHKVYACEVTHQGLSS PVTKSFNRGEC |

The present disclosure is further illustrated through description of the specific embodiments below, but this is not intended to limit the present disclosure. Those skilled in the art may make various modifications or improvements in light of the teachings of the present disclosure without departing from the basic spirit and scope of the present disclosure.

### Example 1: Production of anti-B7H3 antibody-drug conjugate

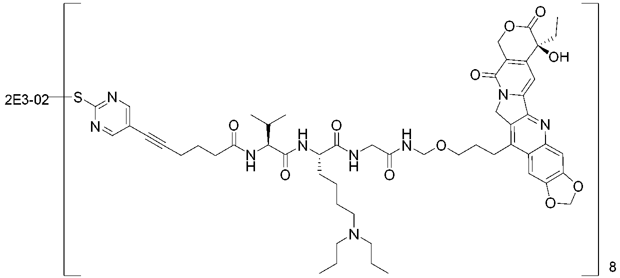

Referring to the production method described in Example 4.1.7.1 of the International Publication No. WO2022170971A1, an anti-B7H3 ADC composition (hereinafter referred to as "ADC1") was produced using a humanised anti-B7H3 antibody (2E3-02).

### Example 2: Pharmaceutical effect testing of antibody-drug conjugate (ADC1) in combination with anti-PD-L1 drug in colon cancer animal model

MC38 cells (Shunran (Shanghai) Biotechnology Co., Ltd.) were genetically modified to express the human B7H3 genes, thereby obtaining B-hB7-H3 MC38 cells. The cells were subcutaneously inoculated into C57BL/6 mice to establish a B-hB7-H3 MC38 tumour cell transplantation tumour model. When the tumours grew to approximately 140 mm³, grouping for animal experiments was performed, a control group (G1: 0.9% sodium chloride injection), monotherapy groups (G2: ADC1 10 mg/kg group, G3: ADC1 3 mg/kg group, G4: anti-PD-L1 3 mg/kg group), and combination therapy groups (G5: group with ADC1 10 mg/kg in combination with anti-PD-L1 3 mg/kg, G6: group with ADC1 3 mg/kg in combination with anti-PD-L1 3 mg/kg) were established respectively, with 8 mice in each group. Dosing was performed according to the regimen in Table 1. The anti-PD-L1 drug was atezolizumab (Biocytogen). At the end of the experiment, therapeutic effects were evaluated according to the tumour growth inhibition (TGI), and tolerability was evaluated according to clinical observations, body weight changes, and mortality of the animals.

**Table 1 Dosing regimen for mice bearing B-hB7-H3 MC38 transplantation tumours**

| **Group** | **Tested product** | **Number of animals (mouse)** | **Dose (mg/kg) ^{a}** | **Route of dosing** | **Dosing frequency ^{b}** | **Number of times of dosing** |
|---|---|---|---|---|---|---|
| G1 | 0.9% sodium chloride injection | 8 | - | i.v. + i.p. | BIW + BIW | 4 + 4 |
| G2 | ADC1 | 8 | 10 | i.v. | BIW | 4 |
| G3 | ADC1 | 8 | 3 | i.v. | BIW | 4 |
| G4 | Anti-PD-L1 | 8 | 3 | i.p. | BIW | 4 |
| G5 | ADC1 + Anti-PD-L1 | 8 | 10 + 3 | i.v. + i.p. | BIW + BIW | 4 + 4 |
| G6 | ADC1 + Anti-PD-L1 | 8 | 3 + 3 | i.v. + i.p. | BIW + BIW | 4 + 4 |

| | | | | | | |
|---|---|---|---|---|---|---|
| Note: a: the dosing volume was calculated at 10 µL/g based on the body weight of the experimental animals, and each drug injection was completed within 10 to 20 seconds. b: BIW refers to administration twice weekly. When the combined treatment groups were dosed on the same day, there was no required sequence or dosing interval between the two drugs. The tumour growth curves are shown in Figure 1, and the specific results are shown in Table 2 below. | | | | | | |

**Table 2 In vivo therapeutic effects of B-hB7-H3 MC38 transplantation tumour model**

| **Group** | **Tested product** | **Tumour volume (mm³) ^{a}** | | | ***p*^{b}** | **Tumour clearance proportion^{c}** |
|---|---|---|---|---|---|---|
| | | **Before dosing** | **Day 22 after grouping for dosing** | **TGI_{TV} (%)** | | |
| G1 | 0.9% sodium chloride injection (-) | 140 ± 3 | 2136 ± 411 | - | - | - |
| G2 | ADC1 (10 mg/kg) | 140 ± 3 | 11 ± 4 | 106.5 | ****<0.0001 | 8/8 |
| G3 | ADC1 (3 mg/kg) | 140 ± 3 | 687 ± 128 | 72.6 | ****<0.0001 | 0/8 |
| G4 | Anti-PD-L1 (3 mg/kg) | 140 ± 3 | 1689 ± 257 | 22.4 | 0.4351 | 0/8 |
| G5 | ADC1 + Anti-PD-L1 (10 + 3 mg/kg) | 140 ± 3 | 5 ± 3 | 106.8 | ****<0.0001 | 8/8 |
| G6 | ADC1 + Anti-PD-L1 (3 + 3 mg/kg) | 140 ± 3 | 374 ± 130 | 88.3 | ****<0.0001 | 2/8 |

| | | | | | | |
|---|---|---|---|---|---|---|
| Note: a: mean ± standard error; b: statistical comparison of the tumour volume in the dosing groups versus the tumour volume in the G1 control group on Day 22 after grouping for dosing, one-way ANOVA analysis and Dunnett's test; ****p <0.0001. c: the tumour clearance proportion was counted according to the results on Day 25. | | | | | | |

The testing results indicated that the TGIs of G2-G6 groups were 106.5%, 72.6%, 22.4%, 106.8%, 88.3%, respectively. The tumour clearance (complete tumour regression) proportions of animals in each group were 8/8, 0/8, 0/8, 8/8, 2/8, respectively. ADC1 at doses of 10 mg/kg and 3 mg/kg as well as ADC1 at the equal doses in combination with anti-PD-L1 (3 mg/kg) respectively have significant inhibitory effects on the growth of subcutaneous transplantation tumours of B-hB7-H3 MC38 tumours, and have a dose dependency. The tumour inhibitory effects of the G6 group with combination dosing were superior to those in the G3 and G4 monotherapy groups, and the tumour inhibitory effects of the G5 group with combination dosing was superior to those in the G4 monotherapy group. Since high-dose ADC1 monotherapy had already achieved the maximum tumour inhibition rate, combination dosing could not further demonstrate a superior antitumour effect of the combination; combination dosing of ADC1 at the lower dose showed improved therapeutic effects compared to the monotherapy in each group.

At the same time, during the dosing period, body weight increased steadily in all groups. The body weight gain in the combined treatment groups was comparable to that in the control group, and the body weight curves substantially overlapped, indicating that neither monotherapy nor combined treatment showed obvious toxicity.

### Example 3: In vivo activity testing of antibody-drug conjugate (ADC) in combination with Anti-PD-L1 antibody

### 1. Experimental Materials

Tested compound: ADC1, an anti-mouse PD-L1 antibody (anti-mPD-L1; BioXcell, BP0101), with saline as the vehicle control.

Experimental cells: B-hB7-H3 CT26. WT cells overexpressing human B7-H3, which were constructed by Biocytogen Pharmaceuticals (Beijing) Co., Ltd.

Experimental animals: female BALB/c mice, 6-8 weeks old, provided by Biocytogen.

### 2. Experimental Regimen

### 2.1. Cell Treatment

The culture conditions for B-hB7-H3 CT26. WT cells were: RPMI 1640 + 10%FBS, cultured in an incubator at 37°C, 5% CO₂. Cells were subjected to conventional digestive treatment and passage twice weekly using trypsin containing EDTA. The cells were washed twice with PBS, and then were centrifuged and resuspended in pre-chilled PBS. Cells were counted using a cell counter, the cell suspension was adjusted to an appropriate concentration, and the cells were inoculated.

### 2.2. Tumour Cell Inoculation

BALB/c mice were acclimatised to the laboratory environment for 3-5 days. Cells were then subcutaneously inoculated into the right dorsal flank of each mouse at 1×10⁶ cells per 0.1 mL PBS. Grouping and dosing were initiated when the mean tumour volume reached about 80-120 mm³.

### 2.3. Animal Dosing and Detection

The enrolled tumour-bearing nude mice were dosed according to the regimen shown in Table 3 below:

**Table 3. Dosing regimen**

| Group | Dosing group | Dose (mg/kg) | Method of dosing | Dosing cycle |
|---|---|---|---|---|
| 1 | Saline | 3 | i.v. | BIW * 4 |
| 2 | ADC1 + anti-mPD-L1 | ADC1 3 mg/kg, anti-mPD-L1 15 mg/kg | i.v. | BIW * 4 |

### 2.4. Measurement of Tumour Volume and Body Weight

The day of the first administration was designated Day 0. A total of four doses were administered, and the diameter of the tumour and body weight were measured regularly. Tumour volume, relative tumour proliferation rate, and relative tumour inhibition rate were calculated, and tumour growth curves were plotted. The formulae were as follows:
The tumour volume (V) was calculated as: V = 1/2 × Llong × Lshort2, wherein Llong and Lshort represent the long diameter and short diameter of the tumour, respectively.

Relative tumour growth inhibition rate, TGI (%), was calculated as follows: TGI (%) = [1 - (mean tumour volume of the treatment group on a given day - mean tumour volume of the treatment group at the start of dosing) / (mean tumour volume of the vehicle control group on the same day - mean tumour volume of the vehicle control group at the start of dosing)] × 100%.

Relative tumour proliferation rate, T/C (%): the calculation formula is as follows: T/C (%) = TRTV/CRTV × 100% (TRTV: RTV of the treatment group; CRTV: RTV of the vehicle control group). RTV (relative tumour volume): the calculation formula is RTV = Vt/V0, wherein V0 is the mean tumour volume obtained by measurements at the time of dosing after grouping (i.e. D0), Vt is the mean tumour volume at a certain measurement, and TRTV and CRTV take data of the same day.

### 3. Experimental Results

The testing results indicated that the antibody-drug conjugate of the present disclosure in combination with an anti-PD-L1 antibody exhibited a relatively significant tumour inhibition effect. During the dosing period, there was no significant reduction in body weight or significant drug toxicity of animals in each group. Specific results are shown in Table 4.

**Table 4. Tumour volume data of B-hB7-H3 CT26.WT model**

| | Average tumour volume (mm³) | | | | | TGI (%) D14 |
|---|---|---|---|---|---|---|
| Group | D0 | D4 | D7 | D11 | D14 | |
| 1 | 92 | 164 | 340 | 566 | 773 | / |
| 2 | 93 | 167 | 232 | 232 | 189 | 85.9% |

The above are merely preferred embodiments of the present invention and are not intended to limit the present invention. Any modifications, equivalent substitutions, improvements and the like made within the spirit and principles of the present invention shall fall within the scope of protection of the present invention. In addition, the technical solutions between the various embodiments may be combined with each other, but must be based on what is achievable by those of ordinary skill in the art; when the combination of the technical solutions is contradictory or cannot be achieved, such combination of the technical solutions should be considered as non-existent and not within the scope of protection claimed by the present invention.

## Claims

1. A method of treating cancer, the method comprising administering to a subject in need thereof an effective amount of:
(a) an anti-B7H3 antibody-drug conjugate, which comprises an anti-B7H3 antibody or antigen-binding fragment thereof and a camptothecin drug, wherein the anti-B7H3 antibody or antigen-binding fragment thereof comprises a heavy chain variable region and a light chain variable region, wherein:
the heavy chain variable region (VH) comprises:
(i) CDR-H1 comprising the amino acid sequence set forth in SEQ ID NO: 1;
(ii) CDR-H2 comprising the amino acid sequence set forth in SEQ ID NO: 2; and
(iii) CDR-H3 comprising the amino acid sequence set forth in SEQ ID NO: 3; and
the light chain variable region (VL) comprises:
(iv) CDR-L1 comprising the amino acid sequence set forth in SEQ ID NO: 4;
(v) CDR-L2 comprising the amino acid sequence set forth in SEQ ID NO: 5; and
(vi) CDR-L3 comprising the amino acid sequence set forth in SEQ ID NO: 6;
wherein the CDRs are determined in accordance with the Kabat definition scheme;
and (b) an additional therapeutic agent, wherein the additional therapeutic agent is selected from one or both of an anti-PD-L1 antibody or antigen-binding fragment thereof and a chemotherapy drug.

2. The method of claim 1, wherein the heavy chain variable region comprises or is an amino acid sequence having at least 85% sequence identity to the amino acid sequence set forth in SEQ ID NO: 7, and the light chain variable region comprises or is an amino acid sequence having at least 85% sequence identity to the amino acid sequence set forth in SEQ ID NO: 8;
preferably, wherein the heavy chain variable region comprises or is the amino acid sequence set forth in SEQ ID NO: 7, and the light chain variable region comprises or is the amino acid sequence set forth in SEQ ID NO: 8;
more preferably, the anti-B7H3 antibody comprises a heavy chain with the amino acid sequence set forth in SEQ ID NO: 9 or with an amino acid sequence having at least 85% sequence identity to SEQ ID NO: 9, and a light chain with the amino acid sequence set forth in SEQ ID NO: 10 or with an amino acid sequence having at least 85% sequence identity to SEQ ID NO: 10;
still more preferably, the heavy chain of the anti-B7H3 antibody comprises the amino acid sequence set forth in SEQ ID NO: 9, and the light chain comprises the amino acid sequence set forth in SEQ ID NO: 10.

3. The method of claim 1 or 2, wherein the anti-B7H3 antibody or antigen-binding fragment thereof is the antibody designated as 2E3 or 2E3-02 in WO2022170971; more preferably, the anti-B7H3 antibody is the antibody designated as 2E3-02.

4. The method of any one of claims 1 to 3, wherein the method comprises administering to the subject in need thereof an effective amount of:
(a) an anti-B7H3 antibody-drug conjugate, wherein the anti-B7H3 antibody-drug conjugate is an antibody-drug conjugate having the structure of the following formula, a pharmaceutically acceptable salt, stereoisomer, or metabolite thereof, or a solvate thereof:
Tb-(L-D)q,
wherein:
Tb is an anti-B7H3 antibody or antigen-binding fragment thereof; preferably, Tb is as defined for the anti-B7H3 antibody or antigen-binding fragment thereof of any one of claims 1 to 3;
L is a linker and has the structure of formula: 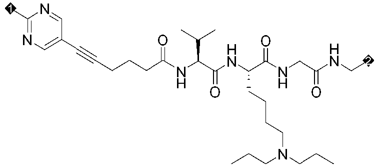
position 1 is linked to Tb, and position 2 is linked to D;
D is a bioactive molecular fragment, preferably a camptothecin drug;
q is selected from any numerical value ranging from 0.1 to 16.0; preferably, q is selected from 1, 2, 3, 4, 5, 6, 7, 8, 9 and 10; more preferably, q is 2, 4, 6 or 8;
and (b) an additional therapeutic agent, wherein the additional therapeutic agent is selected from one or both of an anti-PD-L1 antibody or antigen-binding fragment thereof and a chemotherapy drug.

5. The method of claim 4, wherein the anti-B7H3 antibody-drug conjugate is an antibody-drug conjugate having the structure of formula I, a pharmaceutically acceptable salt, stereoisomer, or metabolite thereof, or a solvate of any of the foregoing: 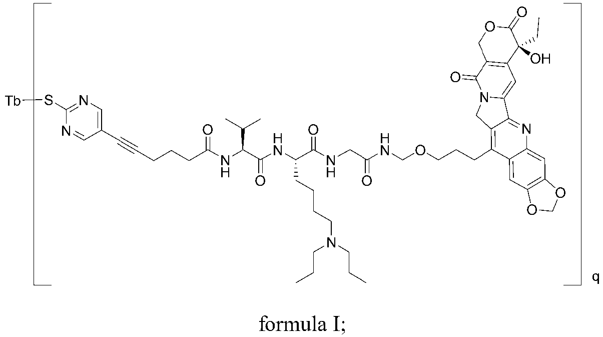 wherein S is a sulphur atom on Tb.

6. The method of any one of claims 1 to 5, wherein the anti-B7H3 antibody-drug conjugate is an antibody-drug conjugate having the structure of formula II, a pharmaceutically acceptable salt, stereoisomer, or metabolite thereof, or a solvate of any of the foregoing: 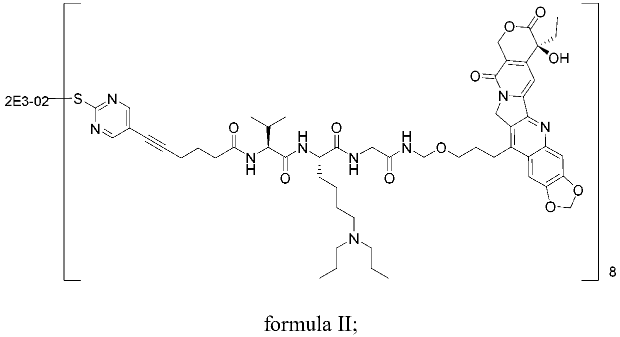
wherein S is a sulphur atom on Tb;
2E3-02 is an anti-B7H3 antibody.

7. The method of any one of claims 1 to 6, wherein the additional therapeutic agent is:
i. an anti-PD-L1 antibody or antigen-binding fragment thereof; or
ii. an anti-PD-L1 antibody or antigen-binding fragment thereof and a chemotherapy drug.

8. The method of any one of claims 1 to 7, wherein the additional therapeutic agent satisfies one or both of the following conditions:
i. the anti-PD-L1 antibody or antigen-binding fragment thereof being selected from one or more of durvalumab, atezolizumab, envafolimab, sugemalimab, adebrelimab, tagitanlimab, socazolimab, and benmelstobart;
ii. the chemotherapy drug being selected from platinum-based drugs; preferably, the platinum-based drugs are selected from one or more of cisplatin, carboplatin, sulfatodiaminocyclohexane platin, nedaplatin, oxaliplatin, lobaplatin, satraplatin, miboplatin, enloplatin, iproplatin, and dicycloplatin.

9. The method of any one of claims 1 to 8, wherein the anti-B7H3 antibody-drug conjugate is administered to the subject at a dose ranging from about 0.1 mg/kg to about 15 mg/kg;
preferably, the anti-B7H3 antibody-drug conjugate is administered to the subject at a dose in the range of 0.1 to 10 mg/kg, 0.2 to 8 mg/kg, 0.3 to 6 mg/kg, 0.4 to 4 mg/kg, or 0.5 to 3 mg/kg of subject body weight;
more preferably, the anti-B7H3 antibody-drug conjugate is administered to the subject at a dose of 0.4 mg/kg, 0.5 mg/kg, 0.6 mg/kg, 0.8 mg/kg, 1.0 mg/kg, 1.2 mg/kg, 1.4 mg/kg, 1.6 mg/kg, 1.8 mg/kg, 2.0 mg/kg, 2.2 mg/kg, 2.4 mg/kg, 2.6 mg/kg, 2.8 mg/kg, 3.0 mg/kg, 4.0 mg/kg, or 5.0 mg/kg of subject body weight.

10. The method of any one of claims 1 to 9, wherein the anti-PD-L1 antibody or antigen-binding fragment thereof is administered to the subject at a flat dose ranging from about 100 mg to about 1500 mg, or at a dose of 2 to 30 mg/kg of subject body weight;
preferably, the anti-PD-L1 antibody or antigen-binding fragment thereof is administered to the subject at a flat dose in the range of 100 to 1400 mg, 100 to 1300 mg, 100 to 1200 mg, 100 to 1100 mg, 100 to 1000 mg, 200 to 1400 mg, 200 to 1300 mg, 200 to 1200 mg, 200 to 1100 mg, 200 to 1000 mg, 300 to 1400 mg, 300 to 1300 mg, 300 to 1200 mg, 300 to 1100 mg, 300 to 1000 mg, 400 to 1400 mg, 400 to 1300 mg, 400 to 1200 mg, 400 to 1100 mg, 400 to 1000 mg, 500 to 1500 mg, 500 to 1400 mg, 500 to 1300 mg, 500 to 1200 mg, 500 to 1100 mg, 500 to 1000 mg, 600 to 1500 mg, 600 to 1400 mg, 600 to 1300 mg, 600 to 1200 mg, 600 to 1100 mg, 600 to 1000 mg, 450 to 550 mg, 550 to 650 mg, 650 to 750 mg, 750 to 850 mg, 550 to 950 mg, 950 to 1050 mg, 1050 to 1150 mg, 1150 to 1250 mg, 1250 to 1350 mg or 1350 to 1450 mg; more preferably, the anti-PD-L1 antibody or antigen-binding fragment thereof is administered to the subject at a flat dose in the range of 600 mg, 900 mg, 1000 mg, 1100 mg, 1200 mg, 1300 mg, 1400 mg or 1500 mg;
still more preferably, the anti-PD-L1 antibody or antigen-binding fragment thereof is administered to the subject at a dose of 2.0 mg/kg, 2.4 mg/kg, 2.8 mg/kg, 3.0 mg/kg, 3.5 mg/kg, 4.0 mg/kg, 4.5 mg/kg, 5.0 mg/kg, 5.5 mg/kg, 6.0 mg/kg, 6.5 mg/kg, 7.0 mg/kg, 7.5 mg/kg, 8.0 mg/kg, 8.5 mg/kg, 9.0 mg/kg, 9.5 mg/kg or 10.0 mg/kg of subject body weight.

11. The method of any one of claims 1 to 10, wherein the chemotherapy drug is a platinum-based drug, and the platinum-based drug is administered to the subject as follows:
i. at a dose in the range of about 50 to 150 mg/m²; preferably, the platinum-based drug is administered to the subject at a dose of 50 mg/m², 70 mg/m², 75 mg/m², 80 mg/m², 100 mg/m², 120 mg/m², or 150 mg/m²;
or
ii. at a dose calculated based on area under the curve (AUC) for each administration, wherein an injection rate of the dose of the administration is 1 to 10 mg/mL/min; preferably, the platinum-based drug is administered to the subject at an injection rate of 3 mg/mL/min, 4 mg/mL/min, 5 mg/mL/min, 6 mg/mL/min, or 7 mg/mL/min.

12. The method of any one of claims 1 to 11, wherein the anti-B7H3 antibody-drug conjugate, the anti-PD-L1 antibody or antigen-binding fragment thereof, or the platinum-based drug is administered in a dosing cycle of 7 to 42 days, for example 21 to 42 days;
preferably:
the anti-B7H3 antibody-drug conjugate is administered at a frequency of once weekly, once every two weeks, once every three weeks, twice every three weeks, once every four weeks, once monthly, once every five weeks, or once every six weeks;
the PD-L1 antibody or antigen-binding fragment thereof is administered at a frequency of once weekly, once every two weeks, once every three weeks, once every four weeks, once monthly, once every five weeks, or once every six weeks;
the platinum-based drug is administered at a frequency of once weekly, once every two weeks, once every three weeks, once every four weeks, once monthly, once every five weeks, or once every six weeks.

13. The method of any one of claims 1 to 12, wherein the anti-B7H3 antibody-drug conjugate and the additional therapeutic agent are administered concurrently, separately, or sequentially.

14. The method of any one of claims 1 to 13, wherein the cancer is selected from a solid tumour;
optionally, the cancer is selected from nasopharyngeal carcinoma, colorectal cancer, non-small cell lung cancer, small cell lung cancer, oesophageal squamous cell carcinoma, prostate cancer, and head and neck squamous cell carcinoma;
preferably, the cancer is selected from nasopharyngeal carcinoma, non-small cell lung cancer (NSCLC), and small cell lung cancer;
more preferably, the non-small cell lung cancer (NSCLC) is selected from squamous NSCLC, adenocarcinoma NSCLC, pulmonary lymphoepithelioma-like carcinoma, and large cell lung cancer; the nasopharyngeal carcinoma is selected from keratinising squamous cell carcinoma of the nasopharynx, non-keratinising nasopharyngeal carcinoma, basaloid squamous cell carcinoma of the nasopharynx, nasopharyngeal carcinoma in situ, and invasive nasopharyngeal carcinoma.

15. The method of claim 14, wherein the subject is a patient with locally recurrent or metastatic nasopharyngeal carcinoma who has previously failed treatment with a platinum-containing regimen; or the subject is a patient with locally recurrent or metastatic nasopharyngeal carcinoma who has not received systemic treatment.

16. The method of claim 14, wherein the subject is a patient with small cell lung cancer who has not received systemic treatment.

17. The method of claim 14, wherein the subject is a patient with locally advanced or metastatic non-small cell lung cancer who has previously failed treatment with a platinum-containing regimen and who is negative for epidermal growth factor receptor (EGFR) sensitising mutations, negative for anaplastic lymphoma kinase (ALK), and/or negative for c-ros proto-oncogene (ROS1); or the subject is a patient with locally advanced or metastatic non-small cell lung cancer who has not received systemic treatment and who is negative for EGFR sensitising mutations, negative for ALK, and/or negative for c-ros proto-oncogene (ROS1).

18. The method of any one of claims 1 to 17, wherein the route of administration of the anti-B7H3 antibody-drug conjugate or the additional therapeutic agent is oral administration, transdermal administration, intravenous injection, subcutaneous injection, or intramuscular injection; preferably, the route of administration of the anti-B7H3 antibody-drug conjugate or the additional therapeutic agent is intravenous injection.

19. The method of any one of claims 1 to 18, wherein one or more therapeutic effects in the subject are improved relative to baseline following administration of the anti-B7H3 antibody-drug conjugate and the anti-PD-L1 antibody or antigen-binding fragment thereof, wherein the one or more therapeutic effects are selected from size of tumour derived from the cancer, objective response rate (ORR), depth of response (DpR), disease control rate (DCR), duration of response (DoR), time to response (TTR), progression-free survival (PFS), and overall survival (OS);
preferably, wherein the size of tumour derived from the cancer is reduced by at least about 10%, at least about 15%, at least about 20%, at least about 25%, at least about 30%, at least about 35%, at least about 40%, at least about 45%, at least about 50%, at least about 60%, at least about 70%, or at least about 80%, relative to the size of tumour prior to administration of the anti-B7H3 antibody-drug conjugate and the anti-PD-L1 antibody or antigen-binding fragment thereof.

20. A pharmaceutical composition comprising an anti-B7H3 antibody-drug conjugate and an additional therapeutic agent, wherein the additional therapeutic agent is selected from one or both of an anti-PD-L1 antibody or antigen-binding fragment thereof and a chemotherapy drug, and the anti-B7H3 antibody-drug conjugate, the anti-PD-L1 antibody or antigen-binding fragment thereof, and the chemotherapy drug are as defined in any one of claims 1 to 19; preferably, the pharmaceutical composition is a pharmaceutical composition for treating cancer, and the cancer is as defined in claim 14;
optionally, the pharmaceutical composition further comprises one or more pharmaceutically acceptable excipients, diluents, or carriers.

21. A kit comprising an anti-B7H3 antibody-drug conjugate and an additional therapeutic agent, wherein the additional therapeutic agent is selected from one or both of an anti-PD-L1 antibody or antigen-binding fragment thereof and a chemotherapy drug, and the anti-B7H3 antibody-drug conjugate, the anti-PD-L1 antibody or antigen-binding fragment thereof, and the chemotherapy drug are as defined in any one of claims 1 to 19;
preferably, the kit comprises a container A and a container B, wherein the container A comprises the anti-B7H3 antibody-drug conjugate as an active ingredient, and the container B comprises the additional therapeutic agent as an active ingredient; and/or the kit is a kit for treating cancer, wherein the cancer is as defined in claim 14.
